**Europäisches Patentamt**

**European Patent Office**

**Office européen des brevets**

(19)

(11) Publication number : **0 404 015 B1**

(12) # EUROPEAN PATENT SPECIFICATION

(45) Date of publication of patent specification :
09.02.94 Bulletin 94/06

(51) Int. Cl.⁵ : **A01N 35/02,** A01N 31/02,
A61L 2/18

(21) Application number : **90111463.7**

(22) Date of filing : **18.06.90**

(54) **A disinfectant composition and a disinfection method using the same.**

(30) Priority : **23.06.89 JP 161270/89**
**01.11.89 JP 286478/89**

(43) Date of publication of application :
**27.12.90 Bulletin 90/52**

(45) Publication of the grant of the patent :
**09.02.94 Bulletin 94/06**

(84) Designated Contracting States :
**AT BE CH DE DK ES FR GB GR IT LI LU NL SE**

(56) References cited :
**EP-A- 0 016 319**
**GB-A- 1 588 648**
**US-A- 4 093 744**

(73) Proprietor : **Nakamura, Junsuke**
**74, Suimoncho**
**Nara-city, Nara (JP)**

(72) Inventor : **Nakamura, Junsuke**
**74, Suimoncho**
**Nara-city, Nara (JP)**

(74) Representative : **Strehl Schübel-Hopf Groening**
**& Partner**
**Maximilianstrasse 54**
**D-80538 München (DE)**

## Description

Background Art

Technical Field

The present invention relates to a disinfectant composition which can be used advantageously in the disinfection of generally confined areas, for example within buildings and vehicles such as buses, ambulance cars, etc., and to a disinfection method using said composition.

Background Art

In the disinfection of a confined area such as the interior of an ambulance car, routine general cleaning must be followed by a time-consuming disinfection work which includes wiping with a swab and disinfectant solution.

The ambulance car, for instance, must be maintained at least at a certain level of cleanliness because its function is to transport patients while they are given initial treatment, and for this purpose, routine disinfection is an important procedure. It is generally acknowledged that the mean viable count of coliform bacteria, staphylococci, general bacteria, fungi and so on is not more than 10 colony-forming units (CFU/10 cm$^2$) and preferably not more than 5 CFU/10 cm$^2$ [Kyukyu Iryo no Kiso to Jissai: 1 Kyukyu Gyomu to Kyukyu Iryo] (The Fundamentals and Practice of Emergency Medicine: 1 Emergency Management and Emergency Medicine, edited by the Emergency Department of the Metropolitan Fire Board of Tokyo, published by Joho Kaihatsu Kenkyusho).

The conventional disinfection procedure comprises using an aqueous dilution of a certain disinfectant such as cresol by manual swab cleaning or spray cleaning with the aid of a hand-operated or electrically-driven sprayer. In some extraordinary or unavoidable cases, a disinfectant solution based on glutaraldehyde, which is known to be an effective sterilant/disinfectant, has been employed. Thus, for example, glutaral is diluted with water to a concentration of about 1 to 2 percent and applied by swab cleaning.

However, the conventional disinfection procedure is not only time-consuming but also inadequate in that all nooks and corners can hardly be reached for thorough disinfection. Moreover, as the diluted disinfectant solution is applied by spraying, the water remains on the treated surface and the desired disinfecting effect is not obtained in corner areas so that no satisfactory result is accomplished.

In addition, when said glutaraldehyde is used for swab cleaning, the irritating and pungent odor persists for a long time. Thus, the odor is in the air for as long as 12 to 24 hours.

It is an object of the present invention to provide a disinfectant composition which can be used advantageously in the disinfection of confined areas such as within the ambulance car under all circumstances, is capable of providing a very high disinfecting effect, can be easily prepared in the field, and does not give off an irritating odor.

It is another object of the invention to provide a disinfection method by which said disinfectant composition can be applied uniformly to all the nooks and corners of such confined spaces.

Summary of the Invention

According to the invention a disinfectant composition is provided, comprising water, a buffer solution, such as an aqueous solution of diethanolamine, 65 to 80 % of alcohol and 2 to 4 % of glutaraldehyde.

In a preferred embodiment the alcohol is selected from ethanol, isopropyl alcohol and denatured ethyl alcohol.

Preferably, the above composition is prepared by blending a basal mixture of said highly concentrated alcohol and a perfume with 10 to 20 volume percent of a 20% (aqueous) solution of glutaraldehyde and 3 to 7 volume percent of a buffer solution such as a 1.0 to 2.0% aqueous solution of diethanolamine.

Thus, since glutaraldehyde has an irritating odor which requires forced ventilation with a fan blower or the like following each cleaning job, a perfume is preferably added as mentioned above for masking this unpleasant odor.

Moreover, if the concentration of glutaraldehyde used in this blending is less than 2%, the disinfectant effect is markedly compromised, while the use of glutaraldehyde over the concentration of 4% is undesirable, for it then gives off an intense irritating odor during cleaning and causes a prolonged carryover of the odor after cleaning.

The concentration of alcohol in the disinfectant composition is preferably in the above-mentioned range of 65 to 80 percent, for both an excess and a shortage of alcohol result in a poor disinfectant effect.

The highly concentrated alcohol mentioned above may be a relatively volatile and biocidally active concentrated alcohol such as ethanol, methanol or of at least 80% concentration isopropyl alcohol, or the corresponding alcohol denatured with some denaturants. For example, there may be mentioned the denatured alcohols obtainable by blending highly concentrated ethanol with denaturants such as methanol, isopropyl alcohol, etc. or, more specifically the so-called methanol-denatured alcohol prepared by adding 5 kg of 95% methanol to each 200 $\ell$ of 95% ethanol.

Since the use of ethanol alone is costly, it is preferable to use a denatured alcohol such as the above-mentioned methanol-denatured alcohol. Isopropyl alcohol of 80 to 90 percent concentration can also be used with advantage from the standpoint of safety.

With the above disinfectant composition, excellent sterilizing and disinfecting effects can be obtained by virtue of the disinfectant glutaraldehyde. Thus, glutaraldehyde is a chemically synthesized substance and has been shown to kill various bacteria, tubercle bacilli, fungi, bacterial spores, and viruses. The use of a solution of this glutaraldehyde as diluted with alcohol produces a sufficient sterilizing and disinfecting effect.

Particularly because said basal concentrated alcohol is used as a diluent for glutaraldehyde, the glutaraldehyde can be well diluted and, in addition, the concentrated alcohol itself exerts an excellent sterilizing and disinfecting effect so that a sufficient effect can be obtained even with a moderate amount of glutaraldehyde (claim 1).

Furthermore, as a buffer solution is included in the disinfectant composition, the pH of the application solution can be maintained at about 7.5 to 8.5 so that the disinfectant effect may last for about 10 days after application. Furthermore, the incorporation of the perfume as a masking agent is useful in cancelling the irritating odor of glutaraldehyde.

Moreover, since the disinfectant composition of the invention can be easily provided by blending said base alcohol, such as concentrated ethanol, isopropyl alcohol or a denatured alcohol prepared by using a methanol or the like as a denaturant, which may be optionally perfumed as mentioned above, with a commercial 20% solution of glutaraldehyde and a buffer solution such as an aqueous solution of diethanolamine, it can be easily prepared in the field and applied as it is.

The disinfection method using the above-mentioned disinfectant composition in accordance with the present invention is characterized in that a disinfectant composition prepared by the above blending procedure is comminuted and sprayed in finely divided form with the aid of a pressurized gas. Generally the above disinfectant composition is applied by spraying about 70 to 80 ml per 25 m$^3$.

With regard to the pressurized gas mentioned above, it is preferable to utilize the pressure of vaporization of liquefied carbon dioxide gas (claim 5). In this case, the disinfectant composition must be sprayed in a mixing ratio well below the dust explosion limit of the alcohol with respect to carbon dioxide gas. This limit is about 0.001 volume percent. Thus, the above ratio is recommended in view of the fact that the lower explosion limit of alcohol in the air is 3.3 volume percent.

The liquefied carbon dioxide gas is sprayed at a gauge pressure of about 3 - 6 kg/cm$^2$.

When sprayed in this manner, the above-mentioned disinfectant composition is atomized in the form of ultramicro particles and can diffuse uniformly into all the nooks and corners throughout the space sprayed therewith. The disinfectant effect of glutaraldehyde can be produced promptly.

Particularly when carbon dioxide gas is used, there is no possibility of chemical reactions with the active ingredient mentioned above, hence there is no fear of changes in properties of the composition, and the glutaraldehyde and highly concentrated alcohol in the disinfectant composition produce an excellent synergistic disinfectant effect.

Furthermore, when carbon dioxide gas is used as a high-pressure gas, the precipitation of disinfectant particles sprayed into the air can be promoted through the specific gravity effect of carbon dioxide gas. Moreover, the use of a large quantity of carbon dioxide gas can fully eliminate the possibility of ignition or explosion in spite of the use of alcohol as the base of the composition. Thus the composition can be sprayed safely even in places where there is heat of fire.

Therefore, said composition can be used to disinfect confined areas such as the interior of an ambulance car efficiently and reliably within a short period of time.

Brief Description of the Drawings

Fig. 1 is a schematic front view of a sprayer which utilizes liquefied carbon dioxide and is to be used for disinfection in the practice of the present invention.

Fig. 2 shows an ambulance car in developments to indicate the points for checking for microorganisms.

## Detailed Description of Preferred Embodiments

(Example 1)

A disinfectant composition containing ethanol (alcohol) in a concentration of 71.25% and glutaraldehyde in a concentration of 4.00% was prepared by blending 850 ml of 95% ethanol, 200 ml of a 20% aqueous solution of glutaraldehyde (Maruishi Pharmaceutical's STERIHYDE L) and 50 ml of a buffer solution, namely a 1.25% aqueous solution of diethanolamine as prepared by dissolving 1.25 ml of 99% diethanolamine in 98.75 ml of distilled water.

This disinfectant composition could remain at pH 8.0 even after the lapse of 10 days without any chemical changes of the active ingredient.

This disinfectant composition was applied to two ambulance cars (car A on service and spare car B) by spraying 40 ml of the composition into each car (12.67 $m^3$ in space volume) through four sites, namely the right and left doors relative to the driver's seat, one side slide door and the rear swing-up door over 40 seconds by means of a sprayer utilizing the pressure of vaporization of liquefied carbon dioxide gas. Then the cars were tightly closed for 20 minutes for effecting disinfection.

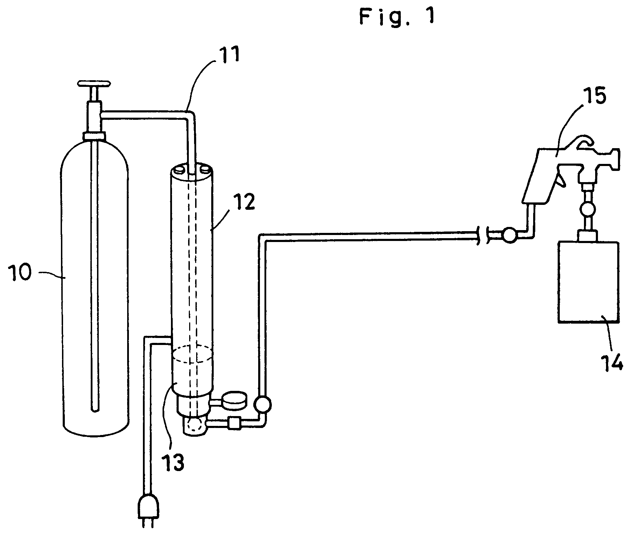

The sprayer mentioned above has, as shown in Fig. 1, for instance, a heater (12) whose temperature is controllable and a pressure adjuster (13) in the discharge line (11) from a carbon dioxide cylinder (10) filled with carbon dioxide gas and equipped with a siphon type delivery mechanism, with a disinfectant composition tank (14) and said discharge line (11) each being connected to a spray gun (15). The liquefied carbon dioxide is warmed for evaporation and then delivery and, at the same time, the pressure of said vaporized gas is utilized to spray the disinfectant composition supplied in the tank (14).

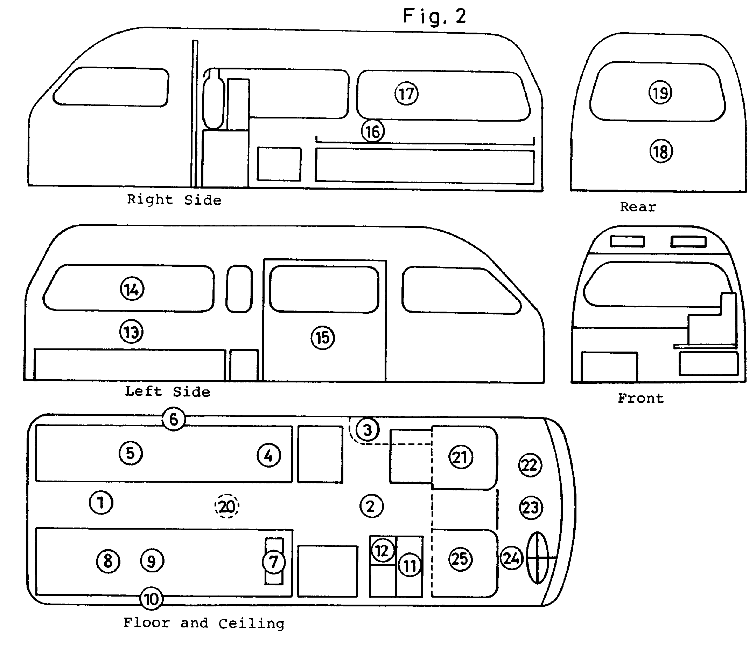

After the above disinfection treatment, four classes of microorganisms, namely coliform bacteria, staphylococci, general bacteria and fungi, were searched for at various source points given circled numbers 1 to 25 in Fig. 2. For the detection of the four groups of microorganisms, the colonies formed on cultivation with the respective specified media were counted. The results thus obtained are shown below in Table 1.

For comparison with the data obtained after the disinfection treatment, the results of the same microorganism counting (before treatment) carried out immediately after car inside cleaning (wiping with wet cloth) are also shown.

(Example 2)

Synthetic methanol-denatured alcohol prepared by adding 24 ml of 95% methanol to 766.0 ml of 95% ethanol was used as the diluent alcohol, and a mixture of 790 ml of said diluent alcohol and 10 ml of a perfume for masking was used as a base. A disinfectant composition having an alcohol concentration of about 76% and a glutaraldehyde concentration of 3% was prepared by blending said base, 150 ml of the same 20% aqueous solution of glutaraldehyde as used in Example 1 (same STERIHYDE L as mentioned above) and 50 ml of a buffer solution, namely a 1% aqueous solution of diethanolamine as prepared by dissolving 1 ml of 99% diethanolamine in 99 ml of distilled water. This disinfectant composition could remain at pH 7.8 to 8.2 even after the lapse of 10 days without any chemical changes of the active ingredient.

This disinfectant composition was applied to two ambulance cars (car A on service and spare car B) in the same manner as in Example 1 by spraying 40 ml of the composition into each car (12.67 $m^3$ in space volume) through three sites, namely two left door sites and the rear swing-up door over 40 seconds, by means of a sprayer utilizing the pressure of vaporization of liquefied carbon dioxide. The cars were then hermetically closed for 20 minutes for achieving the disinfection.

Then, the four classes of microorganisms were searched for at the same 25 points as mentioned above. For comparison, the same microorganism counting was performed before the disinfection treatment. The results thus obtained are shown below in Table 2 in the same manner as in Example 1.

(Example 3)

In this example, use was made of 88.7% isopropyl alcohol as the diluent alcohol in lieu of the synthetic methanol-denatured alcohol used above in Example 2. A mixture of 780 ml of said alcohol and 20 ml of a perfume for masking was used as a base. A disinfectant composition having an alcohol concentration of about 70% and a glutaraldehyde concentration of 3% was prepared by blending said base, 150 ml of a 20% glutaraldehyde solution (same STERIHYDE L as mentioned above) and 50 ml of a buffer solution, namely a 2% aqueous solution of diethanolamine (prepared by dissolving 2 ml of 99% diethanolamine in 98 ml of distilled water).

Like the disinfectant composition of Example 2, this disinfectant composition, too, could remain at pH 7.8 or above even after the lapse of 10 days without any chemical changes of the active ingredient.

This disinfectant composition was used for ambulance car disinfection by spraying it in the same manner as in Example 2, followed by checking for microorganisms. Almost the same results were obtained as those obtained with the disinfectant composition of Example 2.

(Comparative Example)

For comparison, a disinfectant composition was prepared by blending a mixture of 760 ml of 95% ethanol and 200.0 ml of a 20% chlorhexidine gluconate solution (Maruishi Pharmaceuticals's 20% MASKIN solution), 40.0 ml of a 20% glutaraldehyde solution (same STERIHYDE L as mentioned above) and 0.8 ml of 99% diethanolamine (buffer). This disinfectant composition had a pH of 8.0 as measured immediately after preparation thereof. After the lapse of 4 hours, however, the composition became turbid due to the reaction between the MASKIN solution and diethanolamine and showed a pH of 7.2.

The disinfectant composition of this comparative example was applied to the same two ambulance cars (car A on service and spare car B) as used in the foregoing examples by spraying 20 ml of the composition into each car (estimably 9 $m^3$ in space volume) through two sites, namely the door left to the driver's seat and the rear swing-up door, over 20 seconds by means of a sprayer utilizing the pressure of vaporization of liquefied carbon dioxide. The cars were then tightly closed for 15 minutes for effecting disinfection, followed by checking for microorganisms at the same 25 points as mentioned above. The same checking for microorganisms was performed also before treatment. The results thus obtained are shown below in Table 3.

EP 0 404 015 B1

Table 1

Microbial Count: colony forming units (CFU/10 $cm^2$)

| | Source | Ambulance car A (car on service) | | | | | | | | Ambulance car B (spare car) | | | | | | | |
|---|---|---|---|---|---|---|---|---|---|---|---|---|---|---|---|---|---|
| | | General bacteria | | Coliform bacteria | | Staphylococci | | Fungi | | General bacteria | | Coliform bacteria | | Staphylococci | | Fungi | |
| | | Before treatment | After treatment | Before treatment | After treatment | Before treatment | After treatment | Before treatment | After treatment | Before treatment | After treatment | Before treatment | After treatment | Before treatment | After treatment | Before treatment | After treatment |
| 1 | Center of rear floor | 100 | 0 | 0 | 0 | 0 | 0 | 6 | 0 | 0 | 0 | 0 | 0 | 0 | 0 | 8 | 2 |
| 2 | Center of floor adjacent to side door | 100 | 3 | 0 | 0 | 7 | 0 | 10 | 2 | 0 | 0 | 0 | 0 | 0 | 0 | 11 | 3 |
| 3 | Step at side door | 100 | 2 | 0 | 0 | 17 | 0 | 31 | 3 | 0 | 0 | 0 | 0 | 18 | 0 | 15 | 1 |
| 4 | Surface of side seat | 100 | 0 | 0 | 0 | 0 | 0 | 0 | 0 | 0 | 0 | 0 | 0 | 0 | 0 | 0 | 0 |
| 5 | Lid of underside compartment | 100 | 1 | 0 | 0 | 0 | 0 | 0 | 0 | 0 | 0 | 0 | 0 | 0 | 0 | 0 | 1 |
| 6 | Floor of underside compartment | 100 | 0 | 0 | 0 | 11 | 0 | 5 | 0 | 0 | 0 | 0 | 0 | 0 | 0 | 2 | 1 |
| 7 | Underside of patient's pillow | 10 | 0 | 0 | 0 | 2 | 0 | 6 | 0 | 2 | 0 | 0 | 0 | 0 | 0 | 1 | 1 |
| 8 | Surface of stretcher | 0 | 0 | 0 | 0 | 0 | 0· | 5 | 1 | 0 | 0 | 0 | 0 | 0 | 0 | 11 | 0 |
| 9 | Surface of wheel stretcher | 9 | 0 | 0 | 0 | 15 | 0 | 17 | 2 | 0 | 0 | 0 | 0 | 0 | 0 | 4 | 0 |
| 10 | Floor under wheel stretcher | 0 | 0 | 4 | 0 | 0 | 0 | 0 | 0 | 0 | 0 | 0 | 0 | 0 | 0 | 0 | 0 |
| 11 | Surface of oxygen bomb | 4 | 0 | 0 | 0 | 0 | 0 | 0 | 0 | 100 | 4 | 0 | 0 | 0 | 0 | 0 | 0 |
| 12 | Surface of wash basin | 0 | 0 | 0 | 0 | 0 | 0 | 0 | 0 | 0 | 0 | 0 | 0 | 6 | 0 | 4 | 1 |
| 13 | Center of left side wall | 0 | 0 | 0 | 0 | 0 | 0 | 2 | 1 | 0 | 0 | 0 | 0 | 0 | 0 | 0 | 0 |
| 14 | Center of left window glass | 0 | 0 | 0 | 0 | 0 | 0 | 0 | 0 | 0 | 0 | 0 | 0 | 0 | 0 | 0 | 0 |
| 15 | Inner side of side door | 2 | 0 | 0 | 0 | 0 | 0 | 1 | 0 | 0 | 0 | 0 | 0 | 0 | 0 | 0 | 0 |
| 16 | Center of right side wall | 0 | 0 | 0 | 0 | 0 | 0 | 2 | 0 | 0 | 0 | 0 | 0 | 0 | 0 | 0 | 0 |
| 17 | Center of right window glass | 0 | 0 | 0 | 0 | 0 | 0 | 0 | 0 | 0 | 0 | 0 | 0 | 0 | 0 | 0 | 0 |
| 18 | Center of inner surface of rear door | 0 | 0 | 0 | 0 | 0 | 0 | 0 | 0 | 0 | 0 | 0 | 0 | 0 | 0 | 3 | 0 |
| 19 | Center of rear window glass | 0 | 0 | 0 | 0 | 0 | 0 | 0 | 0 | 0 | 0 | 0 | 0 | 0 | 0 | 0 | 0 |
| 20 | Center of ceiling | 0 | 0 | 0 | 0 | 0 | 0 | 0 | 0 | 0 | 0 | 0 | 0 | 0 | 0 | 0 | 0 |
| 21 | Helper's seat | 4 | 1 | 0 | 0 | 0 | 0 | 5 | 2 | 100 | 3 | 0 | 0 | 0 | 0 | 1 | 1 |
| 22 | Telephone set at driver's seat | 10 | 0 | 0 | 0 | 0 | 0 | 0 | 0 | 0 | 0 | 0 | 0 | 0 | 0 | 0 | 0 |
| 23 | Dashboard before helper's seat | 100 | 3 | 0 | 0 | 14 | 0 | 4 | 1 | 0 | 1 | 0 | 0 | 0 | 0 | 3 | 0 |
| 24 | Steering wheel | 0 | 0 | 0 | 0 | 2 | 0 | 0 | 0 | 0 | 0 | 0 | 0 | 0 | 0 | 0 | 0 |
| 25 | Driver's seat | 14 | 1 | 0 | 0 | 0 | 0 | 0 | 0 | 0 | 1 | 0 | 0 | 0 | 0 | 7 | 1 |
| Total | | 753 | 11 | 4 | 0 | 68 | 0 | 94 | 12 | 202 | 9 | 0 | 0 | 24 | 0 | 70 | 12 |
| Mean | | 30.12 | 0.44 | 0.16 | 0 | 2.72 | 0 | 3.76 | 0.48 | 8.08 | 0.36 | 0 | 0 | 0.96 | 0 | 2.80 | 0.48 |
| SD | (standard deviation) | 43.74 | 0.90 | 0.78 | 0 | 5.30 | 0 | 6.84 | 0.85 | 27.02 | 0.98 | 0 | 0 | 3.67 | 0 | 4.20 | 0.76 |
| SE | (standard error) | 8.75 | 0.18 | 0.16 | 0 | 1.06 | 0 | 1.37 | 0.17 | 5.42 | 0.20 | 0 | 0 | 0.73 | 0 | 0.84 | 0.15 |
| AR | (appearance rate %) | 56 | 24 | 4 | 0 | 28 | 0 | 48 | 28 | 12 | 16 | 0 | 0 | 8 | 0 | 48 | 32 |

Table 2

Microbial Count: colony forming units (CFU/10 $cm^2$)

| Source | Ambulance car A (car on service) | | | | | | | | Ambulance car B (spare car) | | | | | | | |
|---|---|---|---|---|---|---|---|---|---|---|---|---|---|---|---|---|
| | General bacteria | | Coliform bacteria | | Staphylococci | | Fungi | | General bacteria | | Coliform bacteria | | Staphylococci | | Fungi | |
| | Before treatment | After treatment | Before treatment | After treatment | Before treatment | After treatment | Before treatment | After treatment | Before treatment | After treatment | Before treatment | After treatment | Before treatment | After treatment | Before treatment | After treatment |
| 1 Center of rear floor | 31 | 3 | 0 | 0 | 63 | 0 | 7 | 4 | 100 | 7 | 0 | 0 | 72 | 0 | 17 | 0 |
| 2 Center of floor adjacent to side door | 100 | 2 | 0 | 0 | 100 | 0 | 11 | 1 | 100 | 7 | 0 | 0 | 100 | 0 | 17 | 0 |
| 3 Step at side door | 100 | 6 | 3 | 0 | 100 | 0 | 12 | 0 | 100 | 14 | 4 | 0 | 100 | 0 | 9 | 2 |
| 4 Surface of side seat | 100 | 3 | 0 | 0 | 1 | 0 | 5 | 1 | 2 | 1 | 0 | 0 | 0 | 0 | 0 | 0 |
| 5 Lid of underside compartment | 12 | 1 | 0 | 0 | 0 | 0 | 11 | 2 | 4 | 0 | 0 | 0 | 0 | 0 | 0 | 0 |
| 6 Floor of underside compartment | 100 | 2 | 0 | 0 | 16 | 0 | 5 | 1 | 100 | 12 | 2 | 0 | 42 | 0 | 22 | 2 |
| 7 Underside of patient's pillow | 1 | 2 | 0 | 0 | 2 | 0 | 6 | 2 | 18 | 1 | 0 | 0 | 0 | 0 | 74 | 0 |
| 8 Surface of stretcher | 26 | 0 | 0 | 0 | 0 | 0 | 2 | 1 | 100 | 7 | 0 | 0 | 16 | 0 | 56 | 4 |
| 9 Surface of wheel stretcher | 22 | 2 | 0 | 0 | 0 | 0 | 0 | 0 | 100 | 4 | 0 | 0 | 4 | 0 | 6 | 4 |
| 10 Floor under wheel stretcher | 100 | 8 | 0 | 0 | 19 | 0 | 21 | 2 | 100 | 10 | 0 | 0 | 10 | 0 | 12 | 1 |
| 11 Surface of oxygen bomb | 2 | 1 | 0 | 0 | 0 | 0 | 0 | 0 | 47 | 0 | 0 | 0 | 0 | 0 | 0 | 2 |
| 12 Surface of wash basin | 0 | 0 | 0 | 0 | 0 | 0 | 16 | 1 | 21 | 1 | 0 | 0 | 0 | 0 | 5 | 0 |
| 13 Center of left side wall | 7 | 1 | 0 | 0 | 0 | 0 | 0 | 0 | 0 | 0 | 0 | 0 | 0 | 0 | 0 | 0 |
| 14 Center of left window glass | 0 | 0 | 0 | 0 | 0 | 0 | 0 | 0 | 0 | 0 | 0 | 0 | 0 | 0 | 0 | 0 |
| 15 Inner side of side door | 3 | 0 | 0 | 0 | 2 | 0 | 1 | 1 | 2 | 0 | 0 | 0 | 0 | 0 | 0 | 0 |
| 16 Center of right side wall | 100 | 0 | 0 | 0 | 0 | 0 | 4 | 0 | 12 | 0 | 0 | 0 | 0 | 0 | 0 | 0 |
| 17 Center of right window glass | 2 | 0 | 0 | 0 | 0 | 0 | 0 | 0 | 100 | 0 | 0 | 0 | 34 | 0 | 19 | 0 |
| 18 Center of inner surface of rear door | 0 | 0 | 0 | 0 | 0 | 0 | 0 | 0 | 0 | 0 | 0 | 0 | 0 | 0 | 0 | 0 |
| 19 Center of rear window glass | 0 | 0 | 0 | 0 | 0 | 0 | 0 | 0 | 0 | 0 | 0 | 0 | 0 | 0 | 0 | 0 |
| 20 Center of ceiling | 0 | 0 | 0 | 0 | 0 | 0 | 0 | 0 | 0 | 0 | 0 | 0 | 0 | 0 | 0 | 0 |
| 21 Helper's seat | 8 | 1 | 0 | 0 | 0 | 0 | 0 | 0 | 22 | 0 | 0 | 0 | 0 | 0 | 0 | 0 |
| 22 Telephone set at driver's seat | 3 | 0 | 0 | 0 | 0 | 0 | 2 | 1 | 100 | 0 | 0 | 0 | 0 | 0 | 0 | 0 |
| 23 Dashboard before helper's seat | 0 | 0 | 0 | 0 | 0 | 0 | 1 | 0 | 100 | 1 | 0 | 0 | 3 | 0 | 11 | 2 |
| 24 Steering wheel | 0 | 0 | 0 | 0 | 0 | 0 | 6 | 2 | 1 | 0 | 0 | 0 | 0 | 0 | 0 | 0 |
| 25 Driver's seat | 100 | 1 | 0 | 0 | 0 | 0 | 8 | 1 | 100 | 0 | 0 | 0 | 7 | 0 | 0 | 0 |
| Total | 817 | 33 | 3 | 0 | 303 | 0 | 118 | 20 | 1229 | 65 | 6 | 0 | 388 | 0 | 248 | 17 |
| Mean | 32.68 | 1.32 | 0.12 | 0 | 12.12 | 0 | 4.72 | 0.80 | 49.16 | 2.60 | 0.24 | 0 | 15.52 | 0 | 9.92 | 0.68 |
| SD (standard deviation) | 42.77 | 1.95 | 0.59 | 0 | 28.93 | 0 | 7.38 | 0.98 | 46.13 | 4.19 | 0.86 | 0 | 30.01 | 0 | 17.87 | 1.22 |
| SE (standard error) | 8.55 | 0.39 | 0.12 | 0 | 5.79 | 0 | 1.48 | 0.20 | 9.23 | 0.84 | 0.17 | 0 | 6.00 | 0 | 3.57 | 0.25 |
| AR (appearance rate %) | 68 | 52 | 4 | 0 | 32 | 0 | 64 | 52 | 80 | 44 | 8 | 0 | 40 | 0 | 44 | 28 |

Table 3

Microbial Count: colony forming units (CFU/10 cm$^2$)

| | Source | Ambulance car A (car on service) | | | | | | | | Ambulance car B (spare car) | | | | | | | |
|---|---|---|---|---|---|---|---|---|---|---|---|---|---|---|---|---|---|
| | | General bacteria | | Coliform bacteria | | Staphylococci | | Fungi | | General bacteria | | Coliform bacteria | | Staphylococci | | Fungi | |
| | | Before treatment | After treatment | Before treatment | After treatment | Before treatment | After treatment | Before treatment | After treatment | Before treatment | After treatment | Before treatment | After treatment | Before treatment | After treatment | Before treatment | After treatment |
| 1 | Center of rear floor | 37 | 2 | 0 | 0 | 12 | 0 | 12 | 2 | 100 | 3 | 0 | 0 | 26 | 2 | 14 | 4 |
| 2 | Center of floor adjacent to side door | 45 | 9 | 0 | 0 | 16 | 6 | 11 | 7 | 24 | 0 | 0 | 0 | 0 | 0 | 6 | 1 |
| 3 | Step at side door | 100 | 11 | 44 | 2 | 61 | 5 | 36 | 8 | 41 | 7 | 0 | 0 | 0 | 0 | 12 | 3 |
| 4 | Surface of side seat | 4 | 0 | 0 | 0 | 5 | 1 | 3 | 0 | 3 | 0 | 0 | 0 | 0 | 0 | 7 | 0 |
| 5 | Lid of underside compartment | 13 | 0 | 0 | 0 | 0 | 0 | 0 | 0 | 0 | 0 | 0 | 0 | 1 | 0 | 2 | 0 |
| 6 | Floor of underside compartment | 16 | 0 | 0 | 0 | 0 | 0 | 10 | 0 | 0 | 0 | 0 | 0 | 0 | 0 | 11 | 2 |
| 7 | Underside of patient's pillow | 100 | 0 | 0 | 0 | 0 | 0, | 0 | 0 | 2 | 0 | 0 | 0 | 0 | 0 | 21 | 0 |
| 8 | Surface of stretcher | 100 | 0 | 2 | 0 | 0 | 0 | 4 | 0 | 1 | 0 | 0 | 0 | 0 | 0 | 15 | 5 |
| 9 | Surface of wheel stretcher | 8 | 0 | 0 | 0 | 2 | 0 | 0 | 0 | 1 | 0 | 0 | 0 | 0 | 0 | 3 | 1 |
| 10 | Floor under wheel stretcher | 17 | 0 | 0 | 0 | 0 | 0 | 2 | 1 | 0 | 0 | 0 | 0 | 0 | 0 | 0 | 1 |
| 11 | Surface of oxygen bomb | 11 | 1 | 0 | 0 | 0 | 0 | 0 | 0 | 11 | 0 | 0 | 0 | 0 | 0 | 2 | 0 |
| 12 | Surface of wash basin | 0 | 0 | 0 | 0 | 0 | 0 | 0 | 2 | 5 | 0 | 0 | 0 | 0 | 0 | 12 | 2 |
| 13 | Center of left side wall | 2 | 0 | 0 | 0 | 0 | 0 | 0 | 0 | 0 | 0 | 0 | 0 | 0 | 0 | 0 | 0 |
| 14 | Center of left window glass | 0 | 0 | 0 | 0 | 0 | 0 | 0 | 0 | 4 | 1 | 0 | 0 | 0 | 0 | 0 | 0 |
| 15 | Inner side of side door | 100 | 1 | 0 | 0 | 2 | 0 | 16 | 4 | 0 | 0 | 0 | 0 | 0 | 0 | 0 | 0 |
| 16 | Center of right side wall | 0 | 0 | 0 | 0 | 0 | 0 | 0 | 0 | 0 | 0 | 0 | 0 | 0 | 0 | 4 | 0 |
| 17 | Center of right window glass | 0 | 0 | 0 | 0 | 0 | 0 | 0 | 0 | 0 | 0 | 0 | 0 | 0 | 0 | 0 | 0 |
| 18 | Center of inner surface of rear door | 11 | 0 | 0 | 0 | 0 | 0 | 0 | 1 | 0 | 0 | 0 | 0 | 0 | 0 | 0 | 0 |
| 19 | Center of rear window glass | 4 | 0 | 0 | 0 | 0 | 0 | 0 | 0 | 0 | 0 | 0 | 0 | 0 | 0 | 0 | 0 |
| 20 | Center of ceiling | 0 | 0 | 0 | 0 | 0 | 0 | 0 | 0 | 0 | 0 | 0 | 0 | 0 | 0 | 0 | 0 |
| 21 | Helper's seat | 6 | 0 | 0 | 0 | 0 | 0 | 3 | 0 | 8 | 0 | 0 | 0 | 0 | 0 | 0 | 0 |
| 22 | Telephone set at driver's seat | 4 | 0 | 0 | 0 | 0 | 0 | 1 | 0 | 25 | 0 | 0 | 0 | 0 | 0 | 11 | 2 |
| 23 | Dashboard before helper's seat | 6 | 0 | 0 | 0 | 4 | 0 | 3 | 0 | 4 | 0 | 0 | 0 | 7 | 0 | 4 | 0 |
| 24 | Steering wheel | 11 | 0 | 0 | 0 | 0 | 0 | 1 | 0 | 67 | 0 | 0 | 0 | 6 | 0 | 0 | 0 |
| 25 | Driver's seat | 6 | 0 | 0 | 0 | 5 | 0 | 0 | 0 | 10 | 0 | 0 | 0 | 0 | 0 | 3 | 0 |
| Total | | 601 | 24 | 46 | 2 | 107 | 12 | 102 | 25 | 306 | 11 | 0 | 0 | 40 | 2 | 127 | 21 |
| Mean | | 24.04 | 0.96 | 1.84 | 0.08 | 4.28 | 0.48 | 4.08 | 1.00 | 12.24 | 0.44 | 0 | 0 | 1.60 | 0.08 | 5.08 | 0.84 |
| SD | (standard deviation) | 34.78 | 2.72 | 8.62 | 0.39 | 12.22 | 1.50 | 7.87 | 2.14 | 23.65 | 1.47 | 0 | 0 | 5.28 | 0.39 | 5.97 | 1.38 |
| SE | (standard error) | 6.96 | 0.54 | 1.72 | 0.08 | 2.45 | 0.30 | 1.58 | 0.43 | 4.73 | 0.29 | 0 | 0 | 1.06 | 0.08 | 1.19 | 0.28 |
| AR | (appearance rate %) | 76 | 20 | 8 | 4 | 32 | 12 | 48 | 28 | 60 | 8 | 0 | 0 | 16 | 4 | 60 | 36 |

EP 0 404 015 B1

As indicated by the data given above in Tables 1 to 3, it was found that, as a result of the disinfection treatment in Example 1 as well as in Example 2, the numbers of microorganisms detected at the respective points and the mean number of microorganisms were very markedly reduced as compared with the corresponding data before treatment, the disinfectant effect being such that the values particularly desired in emergency medicine could be fully cleared.

Although the composition of Example 1 gives off an irritating odor characteristic of glutaraldehyde, the time required for the spraying treatment according to the invention is very short, so that forced ventilation by means of a fan following the disinfection treatment can eliminate the residual odor. Hence, the composition of Example 1 is also usable as a disinfectant.

In particular, the disinfectant compositions of Examples 2 and 3 contain a perfume for masking, so that they do not leave the irritating odor of glutaraldehyde and therefore are improved versions of the composition of Example 1 and particularly suited for use.

Although the disinfectant composition for comparison also produced a sufficient disinfectant effect, said composition allowed chemical reactions to occur. After the lapse of 4 hours, the pH became 7.2 and the disinfectant effect decreased in a relatively short period of time; the composition was thus inferior in stability.

As mentioned above, the disinfectant composition according to the invention, when sprayed utilizing the pressure of vaporization of liquefied carbon dioxide, produced a disinfectant effect which was comparable or superior to the effect obtainable by wiping or spraying with any of the conventional active ingredients. The spray treatment required only a short period of time and uniform disinfection treatment could be achieved in all the nooks and corners.

## Claims

1. A disinfectant composition comprising water, a buffer solution, such as an aqueous solution of diethanolamine, 65 to 80 % of alcohol and 2 to 4 % of glutaraldehyde.

2. A disinfectant composition according to claim 1, wherein the alcohol is selected from ethanol, isopropyl alcohol and denatured ethyl alcohol.

3. A process for producing a disinfectant composition according to claim 1 or 2, characterized in that a basal mixture of the highly concentrated alcohol and a perfume is blended with 10 to 20 volume % of a 20 % solution of glutaraldehyde and 3 to 7 volume % of a buffer solution, such as a 1.0 to 2.0 % aqueous solution of diethanolamine.

4. A method of disinfection which comprises spraying a disinfectant composition claimed in any of claims 1 to 3 in finely divided form into a space to be disinfected with the aid of a pressurized gas.

5. A method of disinfection as claimed in claim 4, wherein the pressure of vaporization of liquefied carbon dioxide is utilized for spraying.

6. A method of disinfection as claimed in claim 5, wherein the disinfectant composition is sprayed in a mixing ratio of about 0.001 volume part (%) of the disinfectant composition to 100 volume parts of carbon dioxide gas.

## Patentansprüche

1. Desinfektionsmittel, enthaltend Wasser, eine Pufferlösung, z.B. eine wäßrige Lösung von Diethanolamin, 65 bis 80 % Alkohol und 2 bis 4 % Glutaraldehyd.

2. Desinfektionsmittel nach Anspruch 1, wobei der Alkohol unter Ethanol, Isopropylalkohol und denaturiertem Ethylalkohol ausgewählt ist.

3. Verfahren zur Herstellung eines Desinfektionsmittels nach Anspruch 1 oder 2, dadurch gekennzeichnet, daß ein Grundgemisch aus dem hochkonzentrierten Alkohol und einem Duftstoff mit 10 bis 20 Volumen-% einer 20 %-igen Lösung von Glutaraldehyd und 3 bis 7 Volumen-% einer Pufferlösung, z.B. einer 1,0 bis 2,0 %-igen wäßrigen Lösung von Diethanolamin, vermischt wird.

4. Verfahren zur Desinfektion, umfassend das Einsprühen eines Desinfektionsmittels nach einem der Ansprüche 1 bis 3 in feinverteilter Form in einen zu desinfizierenden Raum unter Hilfe eines Druckgases.

5. Desinfektionsverfahren nach Anspruch 4, wobei der Verdampfungsdruck von verflüssigtem Kohlendioxid zum Sprühen verwendet wird.

6. Desinfektionsverfahren nach Anspruch 5, wobei das Desinfektionsmittel in einem Mischungsverhältnis von etwa 0,001 Volumenteilen (%) des Desinfektionsmittels zu 100 Volumenteilen des Kohlendioxidgases versprüht wird.

## Revendications

1. Composition désinfectante comprenant de l'eau, une solution tampon telle qu'une solution aqueuse de diéthanolamine, 65 à 80 % d'alcool et 2 à 4 % de glutaraldéhyde.

2. Composition désinfectante selon la revendication 1, dans laquelle l'alcool est choisi parmi l'éthanol, l'alcool isopropylique et l'alcool éthylique dénaturé.

3. Procédé de production d'une composition désinfectante selon la revendication 1 ou 2, caractérisé en ce qu'un mélange de base comprenant l'alcool à concentration élevée et un parfum est mélangé avec 10 à 20 % en volume d'une solution à 20 % de glutaraldéhyde et 3 à 7 % en volume d'une solution tampon telle qu'une solution aqueuse à 1,0 à 2,0 % de diéthanolamine.

4. Procédé de désinfection consistant à pulvériser une composition désinfectante telle que revendiquée dans l'une quelconque des revendications 1 à 3, sous une forme finement divisée, dans un espace qui doit être désinfecté à l'aide d'un gaz sous pression.

5. Procédé de désinfection selon la revendication 4, dans lequel on utilise, pour la pulvérisation, la pression de vaporisation du dioxyde de carbone liquéfié.

6. Procédé de désinfection selon la revendication 5, dans lequel la composition désinfectante est pulvérisée dans un rapport de mélange d'environ 0,001 partie en volume (%) de la composition désinfectante pour 100 parties en volume de dioxyde de carbone gazeux.

Fig. 1

Fig. 2